# EUROPEAN PATENT APPLICATION

(11) **EP 2 189 108 A1**
(43) Date of publication of application: **26.05.2010**
(21) Application number: 08827233.1
(22) Date of filing: 12.08.2008
(51) Int. Cl.: A61B 3/12

(54) **MEIBOMIAN GLAND OBSERVING DEVICE**

(30) Priority: 16.08.2007 JP 2007212030; 30.04.2008 JP 2008118138
(71) Applicant: Amano, Shiro, Tokyo 168-0081 (JP); Arita, Reiko, Tokyo 112-0002 (JP); Kamoto, Yoshinori, Osaka 532-0004 (JP)
(72) Inventor: Amano, Shiro, Tokyo 168-0081 (JP); Arita, Reiko, Tokyo 112-0002 (JP); Kamoto, Yoshinori, Osaka 532-0004 (JP)
(74) Representative: Zimmermann & Partner
(86) International application number: PCT/JP2008/064447
(87) International publication number: WO 2009/022692

(57) **Abstract**

The present invention relates to a device for observing the meibomian glands enables a ready observation necessary for examination without a direct contact of an examination probe to the eyelid and an observation of all of the upper and lower meibomian glands in a relatively short time. A device for observing the meibomian glands, comprising: a light source radiating light containing infrared rays, a visible light-cutting filter that is opaque to visible light but passes at least a portion of infrared rays, an optical system for transmitting light from the light source that has passed through the visible light-cutting filter to the site being observed, an optical system for transmitting light from the site being observed to an infrared camera, an infrared camera, and a display for converting an image picked up by the infrared camera to a visible image and displaying the visible image; wherein the site being observed is a portion comprising the meibomian glands of an eyelid turned inside out, light from the light source is irradiated onto the site being observed, and light reflecting off the site being observed is picked up by the infrared camera.

## Description

### [Cross-reference to related patent applications]

The present application claims priority under Japanese Patent Application 2007-212030, filed on August 16, 2007, and Japanese Patent Application 2008-118138, filed on April 30, 2008, the entire contents of which are hereby incorporated by reference.

### Technical Field

The present invention relates to a device for observing the meibomian glands, and more particularly, to a device permitting observation of the meibomian glands by irradiating light from a light source onto the outer surface of the facial meibomian glands with the eyelids turned inside out.

### Background Art

The meibomian glands secrete tear lipids and form a thin layer of oil, thereby preventing excessive evaporation. Meibomian gland dysfunction ("MGD" hereinafter) is one of the main causes of dry eye syndrome, and may, in some cases, cause tear instability, impair the keratoconjunctival epithelium, or cause chronic blepharitis, contact lens intolerance, or giant papillary conjunctivitis.

Irrespective of clinical severity and the number of persons afflicted, the subjective symptoms of patients often do not correspond to clinical observations. There is still no official basis for diagnosing MGD that is recognized throughout the world. Current objective methods for diagnosing MGD include observing the eyelids with a slit lamp, observing the keratoconjunctival epithelium, meibometry, analysis of the components of lipids secreted by the meibomian glands, and meibography.

Among these, meibography is the only method permitting the observation of morphological change of the meibomian glands by illumination of the meibomian glands from the skin side. The meibomian glands are located within the upper and lower eyelids. Thus, their observation requires that both the upper and lower eyelids be turned inside out and light be passed through the interior of the eyelid from the rear of the eyelid to illuminate it ("Dry Eye Diagnosis PPP" (Stratified diagnosis of the ocular surface "Meibography") Medical View Co., Ltd., May 1, 2002, pp. 72-75, Nonpatent Document 1). (The contents of Nonpatent Document 1 are hereby incorporated in their entirety as reference.)

This method was developed about 30 years ago, and has been gradually improved. However, it has not become widespread because a certain degree of experience and skill are required of the person conducting the examination, the patient experiences a strongly unpleasant sensation as the examination probe comes in direct contact with the eyelids, and the like. Additionally, conventional probes are only able to illuminate a small area and time is required to observe the upper and lower eyelids in their entirety.

Japanese Unexamined Patent Publication (KOKAI) No. 2004-236727 (Patent Document 1) describes an example of an observation device. (Patent Document 1 is hereby incorporated in its entirety as reference.) This device is equipped with a handle member having a means of guiding light from a light source, and an illuminating part that guides light from the light-guiding means to a narrow tubular member extending forward from the tip of the handle portion, irradiating the light toward the lateral surface of the member. The illuminating part is shaped so as to insert behind an eyelid that has been turned inside out and support it in a reversed state. The illuminating part is placed behind the eyelid that has been turned inside out, supporting that portion and illuminating the meibomian glands of the eyelid that has been turned inside out from the rear. [Nonpatent Document 1] "Dry Eye Diagnosis PPP" (Stratified diagnosis of the ocular surface "Meibography"), Medical View Co., Ltd., May 1, 2002, pp. 72-75. [Patent Document 1] Japanese Unexamined Patent Publication (KOKAI) No. 2004-236727

Methods employing meibography were developed about 30 years ago and have been gradually improved. However, they have not become widespread because solutions have not been found for problems such as the requirement of a certain degree of experience and skill in the person conducting the examination, the fact that the patient experiences a strongly unpleasant sensation as the examination probe comes in direct contact with the eyelids, and the like. Additionally, conventional probes are only able to illuminate a small area and time is required to observe the upper and lower eyelids in their entirety.

Nor have the above problems been resolved for the device described in Patent Document 1, namely, the requirement of a certain degree of experience and skill in the person conducting the examination, the fact that the patient experiences a strongly unpleasant sensation as the examination probe comes in direct contact with the eyelids, and the fact that conventional probes are only able to illuminate a small area, requiring time to observe the upper and lower eyelids in their entirety.

The object of the present, devised to solve the above problems, is to provide a device for observing the meibomian glands permitting ready observation as required in an examination, allowing observation without direct contact with the probe used in the examination, and permitting observation of the upper and lower eyelids in their entirety in a relatively short period.

### Disclosure of the Invention

The present inventors conducted a variety of research into solving the above-stated problems, resulting in the successful development of a novel meibography that employs an infrared camera and a visible light-cutting filter, does not involve contact and is thus advantageous to the patient, and permits complete observation in a short period. The present invention was devised on that basis.

The present invention, devised to solve the above-stated problems, is a device for observing the meibomian glands comprising:
a light source radiating light containing infrared rays,
an optical system for transmitting light from the light source to a site being observed,
an optical system for transmitting light from the site being observed to an infrared camera, and
an infrared camera;
   wherein the site being observed is a portion comprising the meibomian glands of an eyelid turned inside out,
a visible light-cutting filter that is opaque to visible light but passes at least a portion of infrared rays is positioned at some point between the light source and the infrared camera; and
light from the light source is irradiated onto the site being observed and light reflecting off the site being observed is picked up by the infrared camera.

The first aspect of the present invention is a device for observing the meibomian glands, comprising:
a light source radiating light containing infrared rays,
a visible light-cutting filter that is opaque to visible light but passes at least a portion of infrared rays,
an optical system for transmitting light from the light source that has passed through the visible light-cutting filter to the site being observed,
an optical system for transmitting light from the site being observed to an infrared camera,
an infrared camera, and
a display for converting the image picked up by the infrared camera to a visible image and displaying the visible image;
   wherein the site being observed is a portion comprising the meibomian glands of an eyelid turned inside out, light from the light source is irradiated onto the site being observed, and light reflecting off the site being observed is picked up by the infrared camera.

The second aspect of the present invention is a device for observing the meibomian glands, comprising:
a light source radiating light containing infrared rays,
an optical system for transmitting light from the light source to a site being observed,
an optical system for transmitting light from the site being observed to an infrared camera,
a visible light-cutting filter that is opaque to visible light but passes at least a portion of infrared rays,
an infrared camera for picking up light from the site being observed that has passed through the visible light-cutting filter, and
a display for converting the image picked up by the infrared camera to a visible image and displaying the visible image;
   wherein the site being observed is a portion comprising the meibomian glands of an eyelid turned inside out, light from the light source is irradiated onto the site being observed, and light reflecting off the site being observed is picked up by the infrared camera.

In the above device for observing the meibomian glands of the present invention, the transition wavelength (midpoint wavelength in the transition region) of the visible light-cutting filter can fall within a range of 700 to 850 nm. Further, the optical system for transmitting light from the light source to the site being observed and the optical system for transmitting light from the site being observed to the infrared camera may comprise beam splitters.

In the device of the present invention, a probe that directly contacts the eyelid and illuminates the meibomian glands from the skin side as is done in conventional meibography is unnecessary. Using the meibography system of the present invention, it is possible to readily observe all of the upper and lower meibomian glands within one minute without imparting any unpleasant sensation whatsoever to the patient.

### Best Mode of Carrying Out the Invention

The present invention is a device for observing the meibomian glands comprising:
a light source radiating light containing infrared rays,
an optical system for transmitting light from the light source to a site being observed,
an optical system for transmitting light from the site being observed to an infrared camera, and
an infrared camera;
   wherein the site being observed is a portion comprising the meibomian glands of an eyelid turned inside out,
a visible light-cutting filter that is opaque to visible light but passes at least a portion of infrared rays is positioned at some point between the light source and the infrared camera; and
light from the light source is irradiated onto the site being observed and light reflecting off the site being observed is picked up by the infrared camera.

The device for observing the meibomian glands of the present invention may further comprise a display for converting the image picked up by the infrared camera into a visible image and displaying it.

A first aspect of the device for observing the meibomian glands of the present invention will be described below. In the first aspect, a visible light-cutting filter is positioned between the light source and the site being observed. The device for observing the meibomian glands of the first aspect comprises:
(a1) a light source radiating light containing infrared rays,
(a2) a visible light-cutting filter that is opaque to visible light but passes at least a portion of infrared rays,
(a3) an optical system for transmitting light from the light source that has passed through the visible light-cutting filter to the site being observed,
(a4) an optical system for transmitting light from the site being observed to an infrared camera,
(a5) an infrared camera, and
(a6) a display for converting the image picked up by the infrared camera to a visible image and displaying the visible image.

### (a1) The light source radiating light containing infrared rays

The light source need only radiate light containing infrared rays. For example, it can be a halogen lamp, mercury lamp, xenon lamp, LED or the like.

### (a2) The visible light-cutting filter that is opaque to visible light but passes at least a portion of infrared rays

In the device of the present invention, the meibomian glands of an eyelid that has been turned inside out constitute the site that is observed. Light from the light source is irradiated onto the surface of the meibomian glands and light reflecting off of the site being observed is picked up by an infrared camera. When visible light is used for observation, there is considerable scattering and the meibomian glands cannot be observed. By contrast, when a visible light-cutting filter is used to eliminate visible radiation, just infrared rays is used to illuminate the site being observed, and observation is conducted with infrared rays, no such scattering problem is produced and good observation of the meibomian glands is possible.

The visible light-cutting filter employed in the present invention need only inhibit scattering of visible light and permit good observation with infrared rays. For example, the visible light-cutting filter may have a transition wavelength (midpoint wavelength in the transition region) falling within a range of 700 to 850 nm, desirably a transition wavelength falling within a range of 720 to 850 nm, preferably a transition wavelength falling within a range of 750 to 850 nm. A visible light-cutting filter with a transition wavelength falling within a range of 750 to 850 nm will be essentially opaque to visible light. By being essentially opaque to visible light, it avoids the problem of scattering visible light.

### (a3) The optical system for transmitting light from the light source that has passed through the visible light-cutting filter to the site being observed and (a4) the optical system for transmitting light from the site being observed to the infrared camera

The slit lamp optical systems that are widely employed in ophthalmologic examinations may be employed as is as the optical system for transmitting light from the light source that has passed through the visible light-cutting filter to the site being observed and the optical system for transmitting light from the site being observed to the infrared camera. The optical system for transmitting light from the light source that has passed through the visible light-cutting filter to the site being observed and the optical system for transmitting light from the site being observed to the infrared camera may comprise beam splitters and/or deflecting mirrors. The beam splitters are used either to separate the light from the light source that is being transmitted to the site being observed and the light from the site being observed that is being transmitted to the infrared camera, or to separate light being transmitted from the site being observed for the infrared camera and for visual observation. The deflecting mirrors are employed to separate light from the optical source that is being transmitted to the site being observed and light from the site being observed that is being transmitted to the infrared camera. These optical systems may also suitably comprise slit apertures, various lenses (such as objective lenses, variable power lenses, and condenser lenses), and the like.

### (a5) The infrared camera

The infrared camera employed in the present invention may be a CCD camera having sensitivity in the near infrared region of 700 to 1,000 nm, for example. Such CCD cameras are employed as devices for conducting examinations on the fundus of eys in ophthalmology. Such CCD cameras having sensitivity in the near infrared region can be employed in the present invention.

### (a6) The display for converting the image picked up by the infrared camera to a visible image and displaying the visible image

Since the infrared camera picks up an infrared image, it is converted to a visible image for observation by doctors and patients. The conversion from infrared image to visible image can be conducted with the conversion function that is built into an infrared camera, for example. The visible image information that is outputted by an infrared camera can be displayed on a monochromatic or color display. The display can be in the form of a cathode-ray tube, liquid crystals, or the like.

A second aspect of the device for observing meibomian glands of the present invention will be described next. In the second aspect, the visible light-cutting filter is positioned between the site being observed and the infrared camera. The device comprises:
(b1) a light source radiating light containing infrared rays,
(b3) an optical system for transmitting light from the light source to a site being observed,
(b4) an optical system for transmitting light from the site being observed to an infrared camera,
(b2) a visible light-cutting filter that is opaque to visible light but passes at least a portion of infrared rays,
(b5) an infrared camera for picking up light from the site being observed that has passed through the visible light-cutting filter, and
(b6) a display for converting the image picked up by the infrared camera to a visible image and displaying the visible image.

### (b1) The light source radiating light containing infrared rays

The light source employed in the second aspect is the same as the light source of (a1) employed in the first aspect.

### (b3) The optical system for transmitting light from the light source to a site being observed

The optical system of (b3) that is employed in the second aspect is identical to the optical system of (a3) that is employed in the first aspect. However, the optical system of (a3) that is employed in the first aspect is for transmitting light from the light source that has passed through the visible light-cutting filter of (a2) to the site being observed, while the optical system of (b3) that is employed in the second aspect is for transmitting light from the light source that has not passed through a visible light-cutting filter to the site being observed.

### (b4) The optical system for transmitting light from the site being observed to an infrared camera

The optical system of (b4) that is employed in the second aspect is the same as the optical system of (a4) that is employed in the first aspect.

### (b2) The visible light-cutting filter that is opaque to visible light but passes at least a portion of infrared rays

The visible light-cutting filter of (b2) that is employed in the second aspect is identical to the visible light-cutting filter of (a2) that is employed in the first aspect. However, in the first aspect, light from the light source of (a1) is passed through the visible light-cutting filter of (a2), and the light that has passed through the visible light-cutting filter of (a2) is sent to the optical system for transmission to the site being observed of (a3). By contrast, the visible light-cutting filter of (b2) that is employed in the second aspect passes light from the site being observed and sends the light that has passed through it to the infrared camera of (b5).

### (b5) The infrared camera for picking up light from the site being observed that has passed through the visible light-cutting filter

The infrared camera of (b5) that is employed in the second aspect is identical to the infrared camera of (a5) that is employed in the first aspect. However, in the first aspect, the light from the optical system of (a4) (where light that is transmitted from the site being observed to the infrared camera) is directly transmitted to the infrared camera. By contrast, in the second aspect, light from the site being observed first passes through the visible light-cutting filter of (b5) before being transmitted to the infrared camera.

### (b6) The display for converting the image picked up by the infrared camera to a visible image and displaying the visible image

The display of (b6) that is employed in the second aspect is identical to the display of (a6) that is employed in the first aspect. Both convert images picked up by an infrared camera into visible images and display them.

In addition to the above, the device of the present invention may comprise a recording device for recording images. The recording device may be a storage medium such as a videotape or hard disk.

The site observed with the device of the present invention is the meibomian glands of an eyelid turned inside out. Light from a light source is irradiated onto the surface of the meibomian glands and light reflecting off the site being observed is picked up by an infrared camera.

In the device of the first aspect of the present invention, light from a light source emitting radiation containing infrared rays is passed through a visible light-cutting filter to cut out the visible light while passing at least a portion of the infrared rays. The light from a light source that has been passed through the visible light-cutting filter is transmitted to the site being observed via an optical system for transmission to the site being observed. The site being observed is the meibomian glands of an eyelid turned inside out. At the site being observed, just the infrared rays is irradiated. Light (infrared rays) reflecting off the site being observed is transmitted to an infrared camera via an optical system for transmission to an infrared camera. The reflected light (infrared rays) that has been transmitted via the optical system is picked up by an infrared camera. The infrared camera converts the image that is picked up to a visible image and displays it on a display. Alternatively, the image information that has been converted to a visible image can be recorded on a storage medium such as a videotape or hard disk, and the recorded image can be displayed on a display.

In the device of the second aspect of the present invention, light from a light source emitting radiation containing infrared rays is transmitted to the site being observed via an optical system for transmission to the site being observed. The site being observed is the meibomian glands of an eyelid turned inside out. Light from a light source containing infrared rays is irradiated onto the site being observed. Light (including infrared rays) reflecting off the site being observed is passed through a visible light-cutting filter via an optical system for transmission to an infrared camera. The visible light-cutting filter cuts out the visible light contained in the reflected light and passes at least a portion of the infrared rays. The reflected light (infrared rays) that has passed through the visible light-cutting filter is picked up by an infrared camera. The image that is picked up by the infrared camera is converted to a visible image and displayed on a display. Alternatively, the image information that has been converted to a visible image can be recorded on a storage medium such as a videotape or hard disk and the recorded image can be displayed on a display.

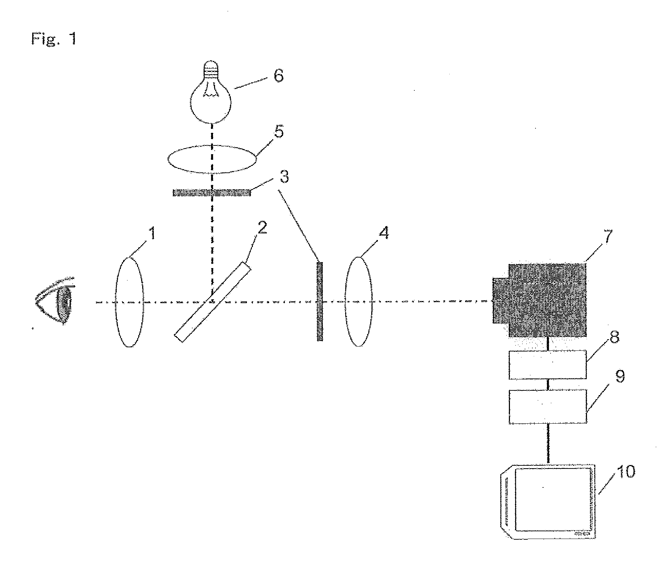

Fig. 1 is a descriptive drawing of an example of the device of the present invention. In the device shown in Fig. 1, visible light-cutting filters 3 are provided at two spots. However, in the actual device, such a filter is provided at only one of these two spots. In the device of the first aspect of the present invention, visible light-cutting filter 3 is positioned between condenser lens 5 and beam splitter (half mirror) 2. In the device of the second aspect of the present invention, visible light-cutting filter 3 is positioned between beam splitter (half mirror) 2 and image pickup lens 4. In both cases, in the course of passing through visible light-cutting filter 3, the visible light is cut out and at least a portion of the infrared rays is passed.

In the device of the first aspect of the present invention, light from a white light source 6 is condensed by a condenser lens 5. The condensed light then passes through visible light-cutting filter 3. The light that has passed through visible light-cutting filter 3 is reflected by a beam splitter (half mirror) 2, passes through objective lens 1, and reaches the meibomian glands of an eyelid that has been turned inside out, which constitute the site being observed. Light that has been irradiated onto and reflected off the meibomian glands sequentially passes through objective lens 1 and beam splitter (half mirror) 2, reaching infrared camera 7 via image pickup lens 4.

In the device of the second aspect of the present invention, light from a white light source 6 is condensed by a condenser lens 5. The condensed light is then reflected by a beam splitter (half mirror) 2, passes through objective lens 1, and reaches the meibomian glands of an eyelid that has been turned inside out, which constitute the site being observed. Light that has been irradiated onto and reflected off the meibomian glands sequentially passes through objective lens 1 and beam splitter (half mirror) 2. Next, it passes through visible light-cutting filter 3. The light that has passed through visible light-cutting filter 3 reaches infrared camera 7 of image pickup lens 4.

In addition to the positions indicated in Fig. 1, visible light-cutting filter 3 can be positioned between beam splitter (half mirror) 2 and the site being observed. For example, it can be positioned between beam splitter (half mirror) 2 and objective lens 1, or between objective lens 1 and the site being observed; the visible light can be cut from the light directed onto the meibomian glands or the light reflecting off the meibomian glands at any position prior to arrival at the infrared camera.

In the device shown in Fig. 1, an adapter 8, video recorder 9, and monitor 10 are connected to infrared camera 7. Adapter 8, video recorder 9, and monitor 10 are connected in series from infrared camera 7. However, at least video recorder 9 and monitor 10 can be connected in parallel from adapter 8.

In the device of the present invention, although not shown in Fig. 1, a slit device (slit aperture and slit aperture projection lens) can be present between condenser lens 5 and beam splitter (half mirror) 2. The slit device can be mounted on a common ophthalmologic diagnostic device. When a slit device is present, light that has been condensed by condenser lens 5 (in the device of the first aspect of the present invention, light that has passed through visible light-cutting filter 3) reaches beam splitter (half mirror) 2 after passing through the slit device.

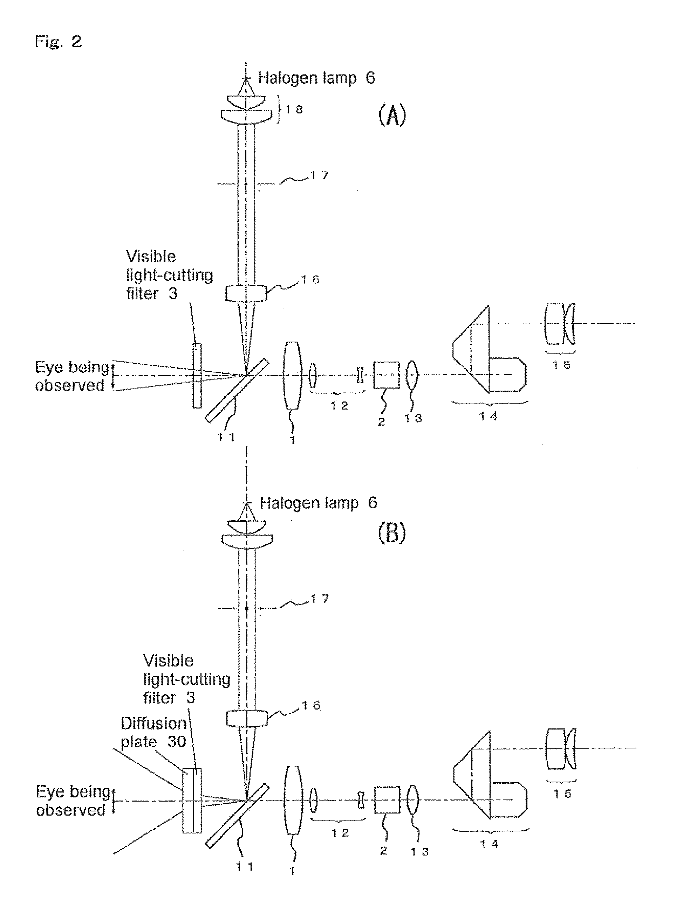

Fig. 2 shows another aspect of the device of the present invention. Fig. 2 is a lateral view of the device. The device for observing the meibomian glands of the aspect shown in Fig. 2(A) comprises, in order from the light source side, a halogen lamp 6, condenser lens used for slit illumination system 18, slit aperture 17, slit aperture projection lens 16, deflecting mirror 11, and visible light-cutting filter 3. Light that has sequentially passed through is irradiated onto the object being observed (subject eye). Light reflecting off the object being observed passes through the periphery of deflecting mirror 11, and is subsequently sequentially transmitted to objective lens 1, variable power lens 12, beam splitter 2, and an infrared camera (not shown). A portion of the light from beam splitter 2 passes through image-forming lens 13, image establishing lens 14, and eyepiece 15, and is observed by the naked eye. In this aspect, as shown in Fig. 2(B), a diffusion plate 30 can be provided to the side of the object being observed side of visible light-cutting filter 3. Providing a diffusion plate 30 affords the advantages of widening the illumination area and permitting observation of a broad range.

In the device of the present invention, the optical system for transmitting light from the light source to the site being observed can comprise an optical fiber. When the optical system for transmitting light from the light source to the site being observed comprises an optical fiber, light from the light source is condensed by a condenser lens and then introduced into the optical fiber. The exit of the optical fiber is positioned in the vicinity of the beam splitter (half mirror) or variable power lens. Light that has been guided by the optical fiber passes via the beam splitter (half mirror) or variable power lens (reflecting off the beam splitter (half mirror) or variable power lens) and reaches to meibomian glands of an eyelid that has been turned inside out, which is the site being observed. In this aspect, the visible light-cutting filter can be positioned, for example, on either the entry side or exit side of the optical fiber, and light that has been guided by the optical fiber can be passed through the visible light-cutting filter before being guided to the beam splitter (half mirror) or variable power lens. Alternatively, visible light-cutting filter 3 can be positioned, not on the exit side of the optical fiber, but between the beam splitter (half mirror) or variable power lens and the infrared camera.

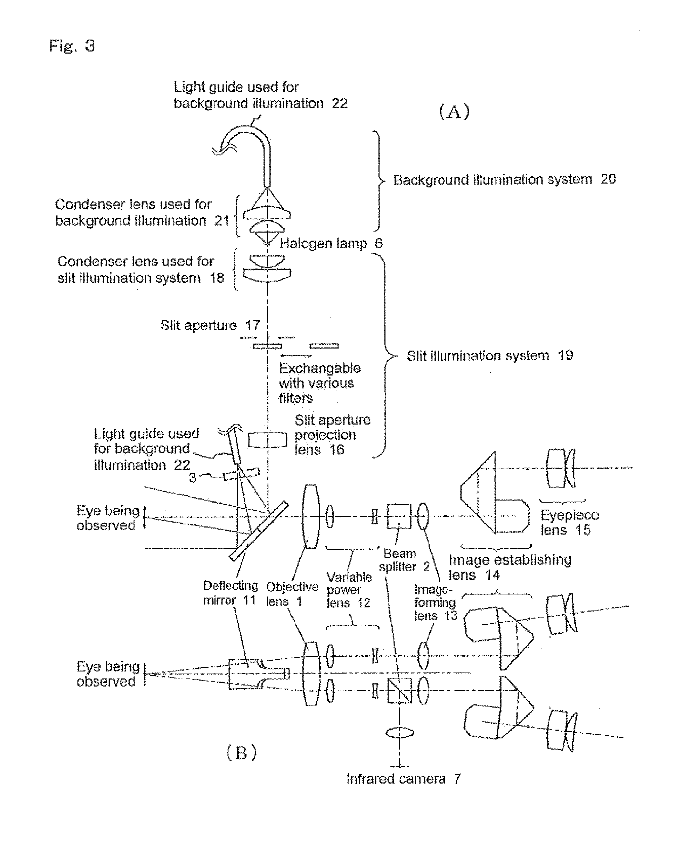

Fig. 3 shows a aspect of the device of the present invention comprising an optical fiber as the optical system for transmitting light from a light source to the site being observed. Fig. 3(A) is a lateral view of the device, and Fig. 3(B) is a plan view of the same. With the exceptions that optical fiber 22 and a corresponding condensing lens 21 are employed to guide light for background illumination, the configuration of the device is the same as that shown in Fig. 2. The device for observing the meibomian glands of the aspect shown in Fig. 3 sequentially comprises, from the light source side, a halogen lamp 6, a condenser lens used for background illumination 21, a light guide used for background illumination 22, and a visible light-cutting filter 3. Light that has sequentially passed through these elements then passes via deflecting mirror 11 and is irradiated onto the object being observed (subject eye). Light reflecting off the object being observed is transmitted to infrared camera 7 and is observed by the naked eye in the same manner as in Fig. 2. Visible light-cutting filter 3 can also be positioned on the entry side of light guide used for background illumination 22.

In the device of the present invention, the optical system for transmitting light from a light source to the site being observed may comprise both an optical system comprising an optical fiber as a light-guiding pathway (in which no slit device is present) and an optical system having the above-described slit device. When an optical system containing optical fiber as a light-guiding pathway and an optical system having a slit device are both present, the portion of the site being observed that is illuminated with light is limited by the slit device in an optical system having a slit device. In contrast, an advantage is afforded in that when light is illuminated via an optical system containing optical fiber as a light-guiding pathway, the illumination of a broader portion of the site being observed with light can be ensured than when a slit device is employed. When the two optical systems are present together, the optical system comprising a slit device can be employed for applications other than observation of meibomian glands.

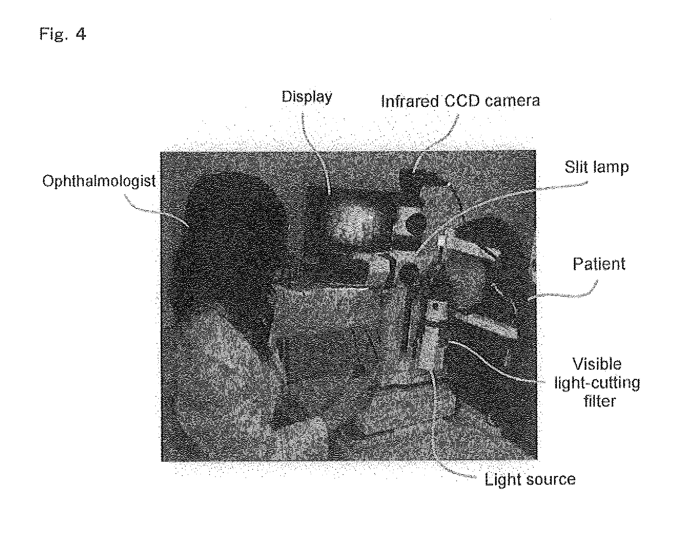

Fig. 4 shows a photograph of an example of the observation device (meibography device) of the present invention. In the example of the observation device of the present invention shown in Fig. 4, a slit lamp (RO5000, Rodenstock) that is widely employed in ophthalmologic medical examinations is utilized as the base. The light source and optical system of the slit lamp are employed, and a visible light-cutting filter (IR-83, Hoya) is installed in the vicinity of the light source (between the light source and the optical system for transmission to the site being observed). An infrared CCD video camera (XC-EI50, Sony) is installed forward of the optical system for transmitting light (infrared rays) reflecting off the site being observed to the infrared camera. A monitor (PVM-1455MD, Sony) for displaying the image (has been converted from infrared image to visible image) from the infrared CCD video camera (XC-EI50, Sony), a camera adapter (DC700, Sony), and a video cassette recorder (SVO-9500MD, DC-700, Sony) are also included.

The term "slit lamp" refers to a microscope employed by ophthalmologists during medical examinations. In the meibography device of the present invention, a visible light-cutting filter passing 750 to 2,800 nm infrared light can be built into the illumination system of the slit lamp and the eyelid illuminated only by infrared rays. A small infrared CCD video camera was also installed above the slit lamp. The filter and camera made it possible to observe with infrared rays the meibomian glands, which were difficult to observe with the diffuse light of a normal slit lamp.

The image from the infrared CCD video camera can be observed at a suitable magnification, for example. In the example shown in Fig. 4, the image is observed on the screen of the monitor at 12-fold magnification. The contrast gain of the monitor and the magnification can be increased to observe in greater detail the acini of the meibomian glands of the pathologyically changed area. Further, a video cassette recorder or the like can be used to record pictures of the meibomian glands of patients. When needed, they can be rewound on site and used to explain the pathosis to the patient.

There are two main advantages to this device. The first is that it can be readily assembled in any ophthalmologic clinic by mounting the above-described visible light-cutting filter, infrared CCD video camera, and monitor (and, as needed, camera adapter and video cassette recorder) on one of the slit lamps that are widely present and can be used by any ophthalmologist to medically examine patients.

The second is that there is no need for the unpleasant light source probe that comes into direct contact with the eyelid and illuminates the meibomian glands from the skin side, which is conceivably why conventional meibography is not found in a large number of facilities. Further, the visible light-cutting filter can be built into the same places and used in the same manner as a blue filter (when fluorescein dye solution is employed) for observing the keratoconjunctival epithelium and the stability of tears. Thus, there is also an advantage in that the person conducting the examination can employ the same slit lamp as in an ordinary medical examination to observe the eyelids and keratoconjunctiva, and then turn a knob with one finger to conduct meibography.

### [Industrial Applicability]

The device for observing the meibomian glands is useful in the field of ophthalmologic examination devices.

### [Brief Description of the Drawings]

[Fig. 1] Fig. 1 is a descriptive drawing of an example of the observation device (meibography device) of the present invention.
[Fig. 2] Fig. 2 is a descriptive drawing of an example of the observation device (meibography device) of the present invention.
[Fig. 3] Fig. 3 is a descriptive drawing of an example of the observation device (meibography device) of the present invention.
[Fig. 4] Fig. 4 is a photograph of an example of the observation device (meibography device) of the present invention.

### [Key to the Numbers]

- 1.: Objective lens
- 2.: Beam splitter (half mirror)
- 3.: Visible light-cutting filter
- 4.: Image pickup lens
- 5.: Condenser lens
- 6.: White light source (halogen lamp)
- 7.: Infrared camera
- 8.: Adapter
- 9.: Video recorder
- 10.: Monitor
- 11.: Deflecting mirror
- 12.: Variable power lens
- 13.: Image-forming lens
- 14.: Image establishing lens
- 15.: Eyepiece lens
- 16.: Slit aperture projection lens
- 17.: Slit aperture
- 18.: Condenser lens used for slit illumination system
- 19.: Slit illumination system
- 20.: Background illumination system
- 21.: Condenser lens used for background illumination
- 22.: Light guide (optical fiber) used for background illumination
- 30.: Diffusion plate

## Claims

1. A device for observing the meibomian glands comprising:
a light source radiating light containing infrared rays,
an optical system for transmitting light from the light source to a site being observed,
an optical system for transmitting light from the site being observed to an infrared camera, and
an infrared camera;
wherein the site being observed is a portion comprising the meibomian glands of an eyelid turned inside out,
a visible light-cutting filter that is opaque to visible light but passes at least a portion of infrared rays is positioned at some point between the light source and the infrared camera; and
light from the light source is irradiated onto the site being observed and light reflecting off the site being observed is picked up by the infrared camera.

2. The device for observing the meibomian glands according to claim 1, wherein the device further comprises a display for converting an image picked up by the infrared camera to a visible image and displaying the visible image

3. The device for observing the meibomian glands according to claim 1 or 2, wherein the optical system for transmitting light from the light source to the site being observed comprises an optical fiber.

4. The device for observing the meibomian glands according to claim 3, wherein the visible light-cutting filter is positioned on either an entry side or exit side of the optical fiber.

5. A device for observing the meibomian glands, comprising:
a light source radiating light containing infrared rays,
a visible light-cutting filter that is opaque to visible light but passes at least a portion of infrared rays,
an optical system for transmitting light from the light source that has passed through the visible light-cutting filter to the site being observed,
an optical system for transmitting light from the site being observed to an infrared camera,
an infrared camera, and
a display for converting an image picked up by the infrared camera to a visible image and displaying the visible image;
wherein the site being observed is a portion comprising the meibomian glands of an eyelid turned inside out, light from the light source is irradiated onto the site being observed, and light reflecting off the site being observed is picked up by the infrared camera.

6. A device for observing the meibomian glands, comprising:
a light source radiating light containing infrared rays,
an optical system for transmitting light from the light source to a site being observed,
an optical system for transmitting light from the site being observed to an infrared camera,
a visible light-cutting filter that is opaque to visible light but passes at least a portion of infrared rays,
an infrared camera for picking up light from the site being observed that has passed through the visible light-cutting filter, and
a display for converting an image picked up by the infrared camera to a visible image and displaying the visible image;
wherein the site being observed is a portion comprising the meibomian glands of an eyelid turned inside out, light from the light source is irradiated onto the site being observed, and light reflecting off the site being observed is picked up by the infrared camera.

7. The device for observing the meibomian glands according to anyone of claims 1-6, wherein the visible light-cutting filter exhibits transition wavelength (midpoint wavelength in the transition region) in a range of 700 to 850 nm.

8. The device for observing the meibomian glands according to anyone of claims 1-7, wherein the optical system for transmitting light from the light source to the site being observed and the optical system for transmitting light from the site being observed to the infrared camera comprise a beam splitter and/or a deflecting mirror.
